# EUROPÄISCHE PATENTSCHRIFT

(11) **EP 2 198 824 B1**
(45) Veröffentlichungstag und Bekanntmachung des Hinweises auf die Patenterteilung: **13.02.2013**
(21) Anmeldenummer: 08022275.5
(22) Anmeldetag: 22.12.2008
(51) Int. Cl.: A61K 6/00, A61K 6/083

(54) **Polymerisierbares Dentalmaterial mit Initiatorsystem**
Polymerizable dental material with initiator system
Matériel dentaire polymérisable doté d'un système d'initiateur

(43) Veröffentlichungstag der Anmeldung: 23.06.2010
(73) Patentinhaber: Ernst Mühlbauer GmbH & Co.KG, 25870 Norderfriedrichskoog (DE)
(72) Erfinder: Neffgen, Stephan, 22459 Hamburg (DE); Hauser, Karsten, 22529 Hamburg (DE); Ziegler, Silke, 22529 Hamburg (DE)
(74) Vertreter: Glawe, Delfs, Moll

(56) Entgegenhaltungen:
- WO-A-02/092023
- WO-A-2008/000313
- DE-A1- 19 928 238

## Beschreibung

Die Erfindung betrifft ein wenigstens zwei flüssige oder pastenförmige Komponenten aufweisendes selbsthärtendes radikalisch polymerisierbares Dentalmaterial. Eine erste Komponente des Dentalmaterials enthält das Salz einer CH-aciden Verbindung und eine weitere Komponente ein Peroxid. Beide Komponenten enthalten (vorzugsweise bei Raumtemperatur) radikalisch polymerisierbare Monomere. Das Dentalmaterial eignet sich besonders für Dentalkleber, Zahnfüllungsmaterialien, Dentalzemente sowie Zahnverblendschalen, provisorische und dauerhafte Kronen- und Brückenmaterialien.

Ein genereller Nachteil radikalisch polymerisierbarer Dentalmaterialien ist ihre begrenzte Lagerstabilität. Speziell Initiatoren enthaltende Pasten und Flüssigkeiten härten oftmals aus bevor sie zu Anwendung kommen oder das Erhärtungsverhalten ändert sich derart, dass das Material für die klinische Anwendung unbrauchbar ist bzw. dass gar keine Erhärtung eintritt. Problematisch sind vor allem auch Änderungen des Erhärtungsverhaltens, die zu verschlechterten Eigenschaften des erhärteten Materials führen. Diese Schwierigkeiten treten insbesondere auf, wenn diese Pasten oder Flüssigkeiten über längere Zeit sommerlichen Temperaturen ausgesetzt sind, sei es beim Transport, der Lagerung in Depots oder der Zahnarztpraxis selbst.

Die meisten Dentalmaterialien wie Fissurenversiegler, Haftvermittler, Dentalkleber und Füllungsmaterialien oder Prothesenkunststoffen wie Kronen- und Brückenmaterialien, die durch radikalische Polymerisation erhärten, enthalten als Starter für die Polymerisation chemisch mehr oder weniger stabile Initiatoren, die durch Licht spezieller Polymerisationslampen aktiviert werden und die Polymerisation in Gang setzen. Diese sind vergleichsweise lagerstabil und das Erhärtungsverhalten gut steuerbar.

Da bei einigen Dentalmaterialien eine solche Lichtpolymerisation bspw. durch Unzugänglichkeit der Restauration oder Lichtundurchlässigkeit des Materials selbst erschwert ist, werden diese Materialien selbsthärtend bereitgestellt. Dies trifft bspw. für Materialien zu, die in Wurzelkanäle eingebracht werden oder zum Stumpfaufbau Verwendung finden oder die zur Befestigung lichtundurchlässiger Zahnersatzteile wie Kronen oder Brücken oder Einlagefüllungen dienen oder die prothetischen Materialien selbst.

Als selbsthärtend werden Materialien bezeichnet deren Polymerisation durch chemische Reaktion der zu vermischenden Komponenten des Initiatorsystems ausgelöst wird. Solche Materialien werden als mindestens zwei Komponenten zur Verfügung gestellt.

Die erste Komponente enthält dabei einen Initiator für die Polymerisation und eine zweite Komponente einen mit dem Initiator reagierenden Akzelerator, Aktivator oder Coinitiator.

Im Dentalbereich am weitesten verbreitete, in zwei Komponenten enthaltene Initatorsysteme sind BPO (Dibenzoylperoxid) als Initiator und ein tertiäres aromatisches Amin als Coinitiator. Dieses Initiatorensystem hat den Nachteil, dass sich dieses enthaltende Materialien, insbesondere auch die fertigen Restaurationen, im Verlaufe der Zeit verfärben und sich somit etwa für ästhetisch anspruchsvolle Restaurationen nicht eignen. Zudem sind solche Initiatorsysteme wenig säureverträglich, sodass die Polymerisationsgeschwindigkeit dann gehemmt und/oder schlecht zu steuern ist.

Weitere gebräuchliche Dentalmaterialien insbesondere Haftvermittler und Zemente mit in zwei Komponenten enthaltenen Initatorsystemen enthalten Barbitursäurederivate als Initiator und lösliche Cu(II)-Verbindungen und Cloride als Aktivatoren sowie andererseits Barbitursäurederivate, wie Benzylphenylbarbitursäure und Peroxide, wie Dibenzoylperoxid (BPO) oder Laurylperoxid wie in zahlreichen Veröffentlichungen bspw. in der DE 19818210 A1, DE 10124028 A1, DE 10124029 A1, EP 1066813 B1, US 5264513, WO 03/057792 A2, US 6818682, EP 1825843 oder EP 1839640 beschrieben. Die Barbitursäurederivate dürfen dabei nicht mit bei Raumtemperatur polymerisierbaren Monomeren in Kontakt kommen, da es sonst zur spontanen Polymerisation kommen kann. Sie werden daher immer entweder als Pulverkomponente oder in inerten Dispergiermitteln eingesetzt.

Bevorzugte Dispergiermittel sind Weichmacher, diese schwächen die polymere Struktur eines Dentalmaterials und können aus toxikologischer Sicht problematisch sein.

Die US 5,707,611 beschreibt wässrige Zahnvorbehandlungsmittel, die einen Initiator für die radikalische Polymerisation und eine Säure enthalten. Der Initiator ist insbesondere ein Barbitursäurederivat oder dessen Salz.

Die EP 0923924 A2 beschreibt einen Dentalklebersatz, dessen säuregruppenhaltige Monomere umfassende Komponente Photoinitiatoren und/oder Peroxide aufweisen kann und die zweite Komponente sich auf auf einem Applikator aufgebrachtes Sulfinsäure-und/oder Barbitursäurederivat und/oder dessen Salz beschränkt. Diese Materialien sind bevorzugt dualhärtend.

Die EP 1502569 A1 beschreibt zweiteilige radikalisch polymerisierbare Monomere enthaltende Dentalkleberzusammensetzungen. Der erste Teil der Zusammensetzung enthält säuregruppenhaltige Monomere, tertiäre Hydroperoxide, oxidierende Übergangsmetallverbindungen des Kupfers, Eisens sowie Kobaltsalze, sowie weiterhin anorganische Peroxide. Der zweite Teil der Zusammensetzung enthält säuregruppenfreie Monomere sowie bevorzugt geringe Mengen oder keine säuregruppenhaltige Monomere sowie reduzierende Thioharnstoff-, Sulfinsäure-, Ascorbinsäure- oder (Thio)Barbitursäurederivate oder deren Salze oder Kupfer-, Eisen- und Kobaltsalze. Der Einsatz von Mikroverkapselung wird ebenfalls beschrieben, was die Materialien aber aufwendig in der Herstellung und somit teurer macht.

Solche Systeme sind ebenfalls oft dualhärtend, da die Erhärtungszeiten sonst zu lang und/oder schlecht steuerbar sind.

Thioharnstoff/Hydroperoxid-Systeme werden ebenfalls in DE 102005039590 A1 und der WO 2007/016508 A1 beschrieben.

Die DE 199 28 238 A beschreibt eine lagerstabile zwei Komponenten umfassende radikalisch polymerisierbare Dentalmasse. Die erste Komponente umfasst mindestens ein bi- oder höherfunktionelles ethylenisch ungesättigtes Monomer, mindestens ein monofunktionelles ethylenisch ungesättigtes Monomer und einen Beschleuniger. Die zweite Komponente weist ein Redoxinitiatorsystem auf, das die radikalische Polymerisation auslösen kann, umfassend ein Barbitursäurederivat und/oder ein Malonylsulfamid und einen ein- oder mehrfunktionellen Carbonsäureperoxyester.

Aus der WO 2008/000313 A1 sind lagerstabile radikalisch polymerisierbare Dentalmaterialien bekannt bestehend aus zwei Komponenten. Eine erste Komponente des Dentalmaterials enthält das Salz einer CH-aciden Verbindung und eine weitere Komponente eine spezielle Säure, deren Säurestärke größer ist, als die der CH-aciden Verbindung. Beide Komponenten enthalten radikalisch polymerisierbare Monomere. Durch Zusatz von Coinitiatoren wie Alkylammoniumchlorid und Cu(II)-Verbindungen wird die Polymerisationszeit verkürzt. Das polymerisierte Dentalmaterial weist jedoch ein verbesserungsfähiges Erhärtungsverhalten und eine geringe Biegefestigkeit auf.

Es besteht somit weiterhin der Bedarf an bei Raumtemperatur radikalisch polymerisierenden Dentalmaterialien, die als Zweikomponentensystem, bevorzugt als zwei Pasten, angemischt werden können, was eine blasenfreie automatische Anmischung bspw. mittels statischer Mischer sowie auch eine leichtere Handanmischung ermöglicht, als dies bei Pulver-Flüssigkeitssystemen möglich ist, und die gleichzeitig eine gute Lagerstabilität, Stabilität gegen spontane (thermische) Selbsterhärtung sowie eine gute Toleranz des Initiatorsystems gegenüber stark sauren Inhaltsstoffen, wie polymerisierbare Säuren, die bspw. für Haftvermittler, selbstklebende Zemente oder Kompomere verwendet werden, besitzen.

Es besteht außerdem weiterhin Bedarf an Dentalmaterialien für Restaurationen, die eine gute Farbstabilität und gute mechanische Eigenschaften im erhärteten Zustand aufweisen. Hier ist die in der ISO-Norm 4049 genannte Grenzfestigkeit von 80 MPa im 3-Punkt-Biegeversuch für Okklusionsmaterialien (Typ 1) als ein bevorzugtes erfindungsgemäßes Kriterium zu benennen.

Aufgabe der Erfindung ist es, selbsthärtende Dentalmaterialien bereitzustellen, die eine verbesserte Stabilität gegen spontane Autopolymerisation, ferner vorzugsweise eine verbesserte Farbstabilität und/oder eine verbesserte mechanische Festigkeit aufweisen. Weiterhin sollen die Materialien vorzugsweise eine gute Säuretoleranz aufweisen.

Die Aufgabe wird durch ein Dentalmaterial gemäß den Patentansprüchen gelöst, welches die folgenden Komponenten umfasst:
Komponente A enthaltend:
   i) radikalisch polymerisierbare Monomere ohne Säuregruppe,
   ii) wenigstens ein Peroxid der Formel (1) 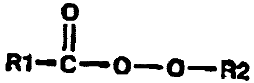
   mit
   R1= H, Alkyl, Aryl, Alkoxy, Aryloxy, oder (R3)(R3)N-;
   R2, R3 gleich oder verschieden ausgewählt aus der Gruppe bestehend aus C(O)-R1, S(O₂)-R1, Alkyl und Aryl.

### Komponente B enthaltend:

iii) radikalisch polymerisierbare Monomere ohne Säuregruppe,
iv) das Salz einer CH-aciden Verbindung, wobei die CH-acide Verbindung eine radikalische Polymerisation auslösen kann.

Die Nachteile der im Stand der Technik bekannten Materialien werden durch das im Anspruch 1 angegebene Material weitgehend behoben.

Weiterhin wurde überraschend gefunden, dass das Salz einer CH-aciden Verbindung (bspw. PBSNa) in der Lage ist, mit den in Anspruch 1 genannten Peroxiden wie bspw. Benzoylperoxid (BPO) bei Raumtemperatur eine radikalische Polymerisation zu bewirken, ohne dass überhaupt eine Säure zugegen ist. Das Material zeigt gutes Erhärtungsverhalten und gute mechanische Eigenschaften.

Die Erfindung hat erkannt, dass überraschenderweise die Kombination eines Salzes einer CH-aciden Verbindung mit bestimmten, im Anspruch definierten Peroxiden ein Initiatorsystem ergibt, das funktionsfähig ist, ohne dass aus dem Salz der CH-aciden Verbindung durch Säurezugabe die nach dem Verständnis des Standes der Technik eigentlich aktive Initiatorverbindung, nämlich die CH-acide Verbindung bzw. Säure, freigesetzt werden müsste.

Erfindungsgemäß ist es somit bevorzugt, dass die Komponente B keine das Salz der CH-aciden Verbindung protonierende Säure enthält. Die Komponente B ist auf diese Weise sehr lagerstabil, da das lagerstabile Salz der CH-aciden Verbindung nicht in die wesentlich reaktionsfähigere und damit weniger lagerstabile CH-acide Verbindung umgewandelt wird.

In der Formel 1 des Anspruchs 1 stellen R1 und R2 organische Reste dar. R1 kann in einer weiteren Variante der Erfindung auch H sein. Die organischen Reste sind und/oder enthalten unverzweigte, verzweigte oder ringförmige Alkyl- sowie Arylgruppen. Die Peroxy-Gruppe in Formel 1 kann auch Teil eines Ringes sein. In diesem Fall sind R1 und R2 über mindestens eine weitere Gruppe miteinander verbunden. Die Peroxy-Gruppe (-C(O)-O-O-) in Formel 1 kann auch mehrfach vorhanden sein. Bevorzugt ist diese dann in R1 und/oder R2 enthalten, besonders bevorzugt ist die Gruppierung R1,2-C(O)-O-O- ein Bestandteil sich wiederholender gleicher oder verschiedener Substituenden an R2 bzw. R1. Es kann dabei bevorzugt sein, dass R1 und/oder R2 ein Polymer ist, bspw. ein Polyether. Die Alkyl- sowie Arylgruppen können Heteroatome wie beispielsweise O, N, S, Si, oder P enthalten. Ferner können die Alkyl- sowie Arylgruppen substituiert sein, beispielsweise durch die Substituenten O, OH, OR3, NR3R3, NR3-C(O)R4, wobei R3 und R4 ebenfalls organische Reste darstellen. Überdies können die organischen Reste partiell oder vollständig ungesättigt sein.

Bevorzugte Peroxide sind Acylperoxide (R1 entspricht darin Alkyl oder Aryl) und Percarbonate (R1 entspricht darin Alkoxy oder Aryloxy). Weiter bevorzugt sind Peroxyester (R1 = Aryl, Alkyl, R2 = Aryl, Alkyl) bzw. Monoperoxycarbonate (R1 = Aryloxy, Alkyloxy, R2 = Aryl, Alkyl). Besonders bevorzugt sind Peroxyester mit R2 = verzweigt Alkyl bzw. quaternärem C-Atom gebunden an die Peroxy-Gruppe. Weiter bevorzugt sind die Diacylperoxide (R2 entspricht darin C(O)-R1, mit R1=Alkyl oder Aryl) und Peroxodicarbonate (R2 entspricht darin C(O)-R1, mit R1=Alkoxy oder Aryloxy). Beispielsweise sind Dibenzoylperoxid (DPO) oder Bis(t-Butylcyclohexyl)-Peroxodicarbonat bevorzugt. Bevorzugt haben die Peroxide eine Einstundenhalbwertstemperatur (T_{1/2}(1h)) von mehr als 60°C, besonders bevorzugt mehr als 90°C. In bestimmen Fällen kann es besonders bevorzugt sein, Peroxide mit einem T_{1/2}(1h)>100°C zu wählen. Die 1-Stunden Halbwertstemperatur ist die Temperatur bei der nach 1 Stunde die Hälfte des Initiators thermisch zerfallen ist.

Geeignete in beiden Komponenten enthaltene bei Raumtemperatur polymerisierbare Monomere sind monofunktionelle und/oder bi- oder höherfunktionelle ethylenisch ungesättigte Monomere bspw. Acrylsäurederivate wie Acrylate, Methacrylate, Cyanacrylate, Acrylamide, Methacrylamide, oder auch bspw. vinyl-, allyl- oder styrolgruppenhaltige Monomere. Beispiele geeigneter Monomere sind Methyl(meth)acrylat, Ethyl(meth)acrylat, n- oder i-Propyl(meth)acrylat, n-, i- oder tert-Butyl(meth)acrylat, Hexyl(meth)acrylat, 2-Ethylhexyl(meth)acrylat, Cyclohexyl(meth)acrylat, Isobornyl(meth)acrylat, Hydroxyethyl(meth)acrylat, Hydroxypropyl(meth)acrylat, Glycerin(meth)acrylat, Glycerin(meth)acrylatester, Glycerin-di(meth)acrylat, Glycerindi(meth)acrylatester, 2-(((Alkylamino)-carbonyl)oxy)ethyl-(meth)acrylate, Allyl(meth)acrylat, Butandioldi(meth)acrylat, Hexandiol-di(meth)acrylat, Decandioldi(meth)acrylat, Dodecandiol-di(meth)acrylat, Ethylenglykoldi(meth)acrylat, Diethylenglykol-di(meth)acrylat, Triethylenglykoldi(meth)acrylat, Tetraethy-lenglykoldi(meth)acrylat, Polyethylenglykoldi(meth)acrylate, Glycerolpropoxytri(meth)acrylat, Trimethylolpro-pantri(meth)acrylat, ethoxylierte und/oder propoxylierte Tri-methylolpropantri(meth)acrylate, Pentaerythrit-tetra(meth)acrylat, Dipentaerythritolhexa(meth)acrylat, Bisphenol-A-di(meth)acrylat, ethoxylierte und/oder propoxylierte Bisphenol-A-di(meth)acrylate, 2,2-Bis-4-(3-(meth)acryloxy-2-hydroxy-propoxy)phenylpropan und davon abgeleitete Verbindungen, Urethan(meth)acrylate (wie z. B. 7,7, 9-Trimethyl-4,13-dioxo-3, 14-dioxa-5,12-diazahexadecan- 1, 16-dioxydimethacrylat), Polyesterurethan(meth)acrylate, Polyester(meth)acrylate, Polycarbonat(meth)acrylate, Polyamid(meth)acrylate, Polyimid(meth)acrylate, Phosphazen(meth)acrylate und Siloxan(meth)acrylate; Ethylvinylether, n- oder i-Propylvinylether, n-, i- oder tert-Butylvinylether, Hexylvinylether, Octylvinlether, Cyclohexylvinylether, Cyclohexyl-3,4-epoxy-1-methylvinylether, Di-methanol-cyclohexylmonovinylether, 1,4-Dimethanol-cyclohexyl-divinylether, Propandioldivinylether, Butandioldivinylether, Hexandioldivinylether, Octandioldivinylether, Decandiolvinylether, Ethylenglykoldivinylether, Diethylenglykoldivinylether, Triethylenglykoldivinylether, Triethylenglykolmonovinylether-mono(meth)acrylsäureester, Polyethylenglykoldivinylether, Tripropylenglykoldivinylether, Glycerintrivinylether, Pentaerythrittetravinylether, 7,7,9-Trimethyl-4,13-dioxo-3,14-dioxa-5,12-diazahexadecan-1,16-dioxydivinylether, Bisphenol-A-divinylether, ethoxylierte und/oder propoxylierte Bisphenol-A-divinylether, Polyestervinylether, Polycarbonatvinylether, Polyacrylatvinylether, Polyamidvinylether, Polyimidvinylether, Polyurethanvinylether, Phosphazenvinylether und Siloxanvinylether ; Glycidyl(meth)acrylat, Dipentendioxid, Bis-(3,4-epoxycyclohexyl)-adipat, Vinylcyclohexenoxide, Vinylcyclohexendioxide sowie difunktionelle 6-Methylen-1,4-dithiepane. Die vinylhaltigen Monomere können dendritische oder hyperverzweigte Strukturen aufweisen.

Die Komponente A kann zusätzlich radikalisch polymerisierbare Monomere mit Säuregruppe enthalten. Bevorzugte säuregruppenhaltige Monomere sind obenstehender Monomere, bevorzugt (Meth)acrylamide und/oder (Meth)acrylester enthaltend eine Säuregruppe. Säuregruppenhaltige Monomere sind bevorzugt nur enthalten, wenn das Material Hafteigenschaften gegenüber dem Zahngewebe aufweisen soll. Geeignete Säuregruppen sind Carbonsäuregruppen oder Carbonsäurederivatgruppen, Sulfonsäuregruppen, Phosphorsäuregruppen, Phosphonsäuregruppen und ähnliche.

Geeignet ist auch (Meth)acrylsäure sowie deren Polymere, die Polymere enthalten bevorzugt zusätzlich polymeriserbare Gruppen, insbesondere (Meth)acrylatgruppen.

Beispiele für geeignete säuregruppenhaltige Monomere sind Methacrylsäure, Acrylsäure, Maleinsäure, Itaconsäure, Maleinsäureanhydrid, 4-Methacryloxyethyltrimellitsäure (4-MET), 4-Methacryloxyethyltrimellitsäureanhydrid (4-META), Additionsprodukte von Mono- oder Dianhydridverbindungen und 2-Hydroxyalkylmethacrylatverbindungen, wie z.B. Pyromellitsäureanhydrid und 2-Hydroxyethylmethacrylat, Pyromellitsäureanhydrid und Glyceroldimethacrylat, 3,3', 4,4'-Benzophenontetracarbonsäuredianhydrid und Hydroxyethylmethacrylat, Phtalsäureanhydrid und Hydroxyethylmethacrylat, Maleinsäureanhydrid und Glyceroldimethacrylat, Methacryloyloxydecylmalonsäure (MAC-10), Maleinsäuremonohemaester, N-Methacryloyl-N',N'-dicarboxymethyl 1,4 diaminobenzen, N-2-Hydroxy-3- methacryloyloxypropyl-N-phenlyglycin, O-Methacryloyltyrosinamid, 4-Methacryloylaminosalicylsäure, Phenylmethacryloxyalkylphosphate wie z.B. Phenylmethacryloxyethylphosphat (Phenly-P), Methacryloyloxyalkyldihydrogenphosphate, wie z.B. Methacryloyloxyethyldihydrogenphosphat (HEMA-P), Methacryloyloxypropyldihydrogenphosphat, Methacryloyloxyhexyldihydrogenphosphat (MHP), Methacryloyloxydecyldihydrogenphosphat (MDP), Glyceriyldimethacrylatphosphat (GPDM), Pentaerythritoltriacrylatphosphat (PENTA-P), Bis(hydroxyethylmethacrylat)phosphat, (Meth)acrylierte Polyacrylsäure, (Meth)acrylamidophosphate, (Meth)acrylamidodiphosphate, (Meth)acrylamidoalkyldiphosphonate, Bismethacrylamidoalkyldihydrogenphosphate, Vinylbenzylphosphonsäure, Vinylbenzoesäure, sowie die Umsetzungsprodukte nukleophiler (Meth)acrylate mit Phosphonsäure- oder Phosphinsäurederivaten wie z. B. P205, POC13 oder PC13.

Zusätzlich können Säuren die keine Monomere darstellen enthalten sein. Beispielhaft genannt seinen Weinsäure, Ethylendiamintetraessigsäure, Zitronensäure, Essigsäure, Trimellitsäure, Trimellitsäureanhydrid, Alkylsulfonsäuren, Arylsulfonsäuren, Schwefelsäure, Salzsäure, Salpetersäure sowie Phosphorsäure.

Die das Salz einer CH-aciden Verbindung enthaltende Komponente B enthält bevorzugt zusätzlich spezielle basische Verbindungen, diese basischen Verbindungen können anorganisch und organischsein. Hierunter sind beispielsweise basische Ionenaustauscher (anorganisch und organisch) und basische Polymere zu verstehen. Bevorzugte basische Verbindungen können Derivate von Aminen, Amidinen, Guanidinen, Polyaminen wie Polyethyleniminen sein. Die Stickstoffatome sind dabei bevorzugt mit Alkyl- oder Arylgruppen vollständig substituiert. Die basischen Verbindungen enthalten besonders bevorzugt radikalisch polymerisierbare Vinylgruppen (basische Monomere).. Die polymerisierbaren Vinylgruppen sind bevorzugt Acryl-, Methacryl, Acrylamid, Methacrylamid- und Styrylgruppen. Solche basischen Monomere sind bevorzugt bspw. N,N-Dimethylaminopropyl(meth)acrylamid, N,N-Dimethylaminoethyl(meth)acrylat oder N,N-Dimethylaminopropyl(meth)acrylat.

Die basische Verbindung ist bevorzugt nur in der Komponente B mit dem CH-aciden Salz bzw. ohne Monomere mit Säuregruppen enthalten. Geeignete Anteile des basischen Monomers in dieser Komponente sind 0,2 bis 5 Gew.-%, bevorzugt 0,5 bis 4 Gew.-%, besonders bevorzugt 1 bis 3 Gew.-%. Geeignete Konzentrationen basischer Monomere im fertig angemischten und/oder polymerisierten Dentalmaterial sind vom Mischungsverhältnis der Komponenten abhängig. Bevorzugt sollte die Konzentration der basischen Monomere dort nicht wesentlich höher als etwa 2 Gew.-% sein. Der Zusatz basischer Verbindungen zur Komponente B erhöht nochmals die Lagerfähig des erfindungsgemäßen Materials, da das CH-acide Salz weiter stabilisiert wird. Es ist in besonderem Maße überraschend, dass die basischen Verbindungen die Fähigkeit des CH-aciden Salzes, als Initiatorkomponente zu wirken, nicht oder nur unwesentlich beeinträchtigen.

Erfindungsgemäß enthält der Harzanteil der Komponenten des polymerisierbaren Dentalmaterials zusammen über 50 Gew.-%, vorzugsweise über 60 Gew.-%, weiter vorzugsweise über 70 Gew.-%, weiter vorzugsweise über 80 Gew.-%, weiter vorzugsweise über 90 Gew.-%, weiter vorzugsweise über 95 Gew.-%, weiter vorzugsweise über 98 Gew.-% radikalisch polymerisierbare Monomere.

Das Salz der CH-aciden Verbindung der Komponente B kann ein Salz der α-Benzoylproprionitrile, α-Cyancarbonsäureester und - amide, cyclischer β-Oxonitrile, β-Diketone, cyclischer β-Diketone, cyclischer β-Oxocarbonsäureester, cyclischer β-Oxolactone, Malonsäure, Malonsäurederivate, Pyrazolderivate, Barbitursäure, Thiobarbitursäure oder Barbitursäure oder Thiobarbitursäurederivate sein. Das Kation des Salzes kann ein ein- oder zweiwertiges Metallion wie ein Alkali-, bspw. Natrium, oder Erdalkaliion sein, ein Gegenion mit einem positiv geladenen Stickstoffatom oder ein Gegenion mit einem positiv geladenen Phosphoratom. Bevorzugt sind Gegenionen mit positiv geladenem Stickstoffatom Ammoniumionen der Formel NR₄⁺, wobei R gleich oder verschieden sein kann und ein H-Atom, eine Alkylgruppe, eine Arylgruppe oder eine Aralkylgruppe, wie z.B. eine Benzylgruppe, darstellt. Alkyl-, Arylalkyl- oder Arylgruppen können mit Heteroatome tragenden Gruppen wie OH-Gruppen oder Ethergruppen substituiert sein. Gegenionen mit positiv geladenem Phosphoratom sind bevorzugt Phosphoniumionen der Formel PR₄⁺, wobei R gleich oder verschieden sein kann und ein H-Atom, eine Alkylgruppe, eine Arylgruppe oder eine Arylalkylgruppe darstellt. Alkyl-, Aralkyl- oder Arylgruppen können mit Heteroatome tragenden Gruppen wie OH-Gruppen oder Ethergruppen substituiert sein.

Weiterhin sind bevorzugt Metallionen, die zum Wechsel der Oxidationsstufe befähigt sind, enthalten. Bevorzugte Metallverbindungen sind die der Übergangselemente wie Eisen und Kobalt. Besonders bevorzugt sind Kupferverbindungen.

Die Metallverbindungen sind bevorzugt in den Komponenten des Dentalmaterials löslich, besonders geeignet sind Kupfer(II)-Salze organischer Säuren.

Die Komponenten können weiterhin verstärkende oder nichtverstärkende Füllstoffe enthalten. Geeignete Füllstoffe sind dem Fachmann bspw. aus der WO 2005/084611 A1 bekannt. Genannt seien hier beispielhaft inerte und/oder reaktive Dentalgläser wie Bariumsilikatglas, Strontiumsilikatglas, Borosilikatglas und Fluoralumosilikatglas oder pyrogene, gefällte oder fossile Kieselsäuren. Bevorzugt werden Mischungen verschiedengroßer Füllstoffpartikel mit Teilchengrößen zwischen 0,001 und 100 µm eingesetzt.

Geeignete Füllstoffe können beispielsweise ausgewählt sein aus der Gruppe bestehend aus mikro- und/oder nanoskaligen Füllstoffen, vorzugsweise Metall-, Halbmetall- oder Mischmetalloxide, Silikaten, Nitriden, Sulfaten, Titanaten, Zirkonaten, Stannaten, Wolframaten, Siliciumdioxiden oder einer Mischung aus diesen Verbindungen oder sphärischen Füllstoffen, Quarzpulvern, Glaspulvern, Glaskeramikpulvern oder eine Mischung aus diesen Pulvern oder gefüllten oder ungefüllten Splitterpolymerisaten und/oder Perlpolymerisaten.

Die Füllstoffe können oberflächenmodifiziert, vorzugsweise organisch oberflächenmodifiziert wie beispielsweise silanisiert sein. Ein oberflächenmodifizierter Füllstoff kann auf seiner Oberfläche funktionelle Gruppen besitzen, die mit den Monomeren chemisch, vorzugsweise radikalisch, reagieren können oder eine hohe Affinität zu der aus den Monomeren gebildeten Polymermatrix haben, wobei der Füllstoff vorzugsweise mit reaktive Acrylat- oder Methacrylatgruppen tragendem Silan oberflächenmodifiziert ist.

Bevorzugte Füllstoffgehalte des erfindungsgemäßen Materials insgesamt liegen zwischen 10 und 90 Gew.-%, vorzugsweise 20 und 80 Gew.-%, weiter vorzugsweise 30 und 70 Gew.-%, weiter vorzugsweise 40 und 60 Gew.-%. Es ist bevorzugt, wenn die Komponenten A und B jeweils ähnliche Gesamtgehalte an Füllstoff besitzen.

Die Komponenten können zusätzlich Initiatoren für die Lichtpolymerisation enthalten, wenn das Material dualhärtend sein soll. Diese sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm und optional durch die zusätzliche Reaktion mit einem oder mehreren Coinitiatoren die Aushärtung des Materials bewirken können. Bevorzugt werden hier Phosphinoxide, Bezoinether, Benzilketale, Acetophenone, Benzophenone, Thioxanthone, Bisimidazole, Metallocene, Fluorone, a-Dicarbonylverbindungen, Aryldiazoniumsalze, Arylsulfoniumsalze, Aryliodoniumsalze, Ferroceniumsalze, Phenylphosphoniumsalze oder eine Mischung aus diesen Verbindungen eingesetzt.

Besonders bevorzugt werden Diphenyl-2,4,6-trimethyl-benzoylphosphinoxid, Benzoin, Benzoinalkylether, Benzildialkylketale, a-Hydroxyacetophenon, Dialkoxyacetophenone, a-Aminoacetophenone, i-Propylthioxanthon, Campherchinon, Phenylpropandion, 5,7-Diiodo-3-butoxy-6-fluoron, (eta-6- Cumol) (eta-5-cyclopentadienyl) eisenhexafluorophosphat, (eta-6-Cumol) (eta-5-cyclopentadienyl) eisentetrafluoroborat, (eta-6-Cumol) (eta-5- cyclopentadienyl) eisenhexafluoroantimonat, substituierte Diaryliodoniumsalze, Triarylsulfoniumsalze oder eine Mischung aus diesen Verbindungen eingesetzt.

Als Coinitiatoren für eine photochemische Aushärtung geeignet sind Borate, organische Phosphite, Diaryliodoniumverbindungen, Thioxanthone, Xanthen, Fluore- ne, Fluorone, a-Dicarbonylverbindungen, kondensierte Polyaromaten sowie tertiäre Amine oder eine Mischung aus diesen Verbindungen. Besonders bevorzugt werden 2-Ethylhexyl-p-(dimethyl-amino)-benzoat und Butyrylcholin-triphenylbutylborat oder eine Mischung aus diesen Verbindungen eingesetzt.

Die Komponenten können weiterhin Stabilisatoren enthalten, wie substituierte und unsubstituierte Hydroxyaromaten, Tinuvine, Terpinene, Phenothiazin, sogenannte HALS (Hindered Amine Light Stabilizers) und/oder Schwermetallfänger wie EDTA; bevorzugte Stabilisatoren sind BHT, butyliertes Hydroxytoluen; Hydrochinon; MEHQ Monomethyletherhydrochinon u.ä.

Durch die erfindungsgemäße Kombination (wie bspw. BPO-Monomerpaste/PBSNa-Monomerpaste bzw. BPO-Monomerflüssigkeit/PBSNa-Monomerflüssigkeit) wird ein Material bereitgestellt, dass eine unerreichte Lagerstabilität bei hervorragender (automatischer) Anmischbarkeit, eine sehr gute Farbstabilität, eine sehr gute Steuerung des Erhärtungsverhaltens und verbesserte mechanische Eigenschaften aufweist.

Bevorzugt wird das Material in Mehrfachkartuschen gelagert und über eine Mischwendel enthaltende Kanüle ausgebracht bzw. appliziert.

Gegenstand der Erfindung ist ferner die Verwendung des polymerisierbaren Dentalmaterials nach einem der vorangehenden Ansprüche als Füllungsmaterial, Stumpfaufbaumaterial, Befestigungsmaterial, Bondingmaterial, provisorisches und permanentes Kronen- und Brückenmaterial, zahntechnischen Werkstoff zur Herstellung von Inlays, Onlays, Verblendschalen, künstlichen Zähnen, Modellmaterialien und Fissuren- bzw. Wurzelkanal-Versiegelungsmaterial.

Das erfindungsgemäße Material kann somit für die prothetische, konservierende und präventive Zahnheilkunde verwendet werden. Bevorzugt kommt es als Zweikomponentenkit zum Einsatz, der automatisch angemischt und appliziert werden kann.

Ein weiterer Gegenstand der Erfindung ist das gehärtete Dentalmaterial, das aus einem erfindungsgemäßen polymerisierbaren Dentalmaterial durch Aushärten erhältlich ist.

Die Erfindung wird anhand nachfolgender Beispiele näher erläutert.

| Folgende Verbindungen kame zum Einsatz: | 2,2-bis[4-(2-Hydroxy-3-Methacryloxypropoxy)phenyl]propan |
|---|---|
| Bis-GMA | |
| TEGDMA | Triethylenglycoldimethacrylat |
| UDMA | 1,6-bis(Methacryloxy-2-Ethoxycarbonylamino)-2,4,4-trimethylhexan |
| GDMA | Glycerindimethacrylat |
| HEMA | 2-Hydroxyethylmethacrylat |
| BTBACl | Benzyltributylammoniumchlorid |
| DHBS | 2,5-Dihydroxybenzoesäure |
| MMHE | Maleinsäure-Mono-2-Methacryloyloxyethylester |
| HEAP | Hydroxyethylacrylat-Phosphorsäureester |
| CuDMA | Kupferdimethacrylat |
| BPO | Dibenzoylperoxid |
| R812S | Aerosil R812S (Degussa) |
| GM 27884-1,5 | Glas GM 27884 in d₅₀=1,5 µm Mahlung |
| TMBSNa | Trimethylbarbitursäure Natriumsalz |
| PBSNa | Phenylbenzylbarbitursäure Natriumsalz |
| N,N-Bis | N,N-Bishydroxyethyl-p-toluidin |
| Aminoester | N,N-Dimethyaminoethylmethacrylat |
| MEMO | Methacryloyloxypropyltrimethoxysilan |

### Herstellungsbeispiel 1 (BAF1, 5sil.)

Es wurde ein Füllstoff BAF1,5sil. hergestellt indem 95 Gewichtsteile GM 27884-1,5 mit 3,8 Gewichtsteilen MEMO und 1,4 Gewichtsteilen Wasser in einem Trockenprozess silanisiert wurden.

### Herstellungsbeispiel 2 (Harz45/55)

Es wurde eine Harzmischung Harz45/55 hergestellt indem 55 Gewichtsteile Bis-GMA mit 45 Gewichtsteilen TEGDMA in einem Laborgefäß mit einem Überkopfrührer bis zur homogenen Lösung verrührt wurden.

### Herstellungsbeispiel 3 (BTBACl-Lösung)

Es wurde eine homogene BTBACl-Lösung hergestellt indem 47 Gewichtsteile BTBACl mithilfe eines Magnetrührers in 53 Gewichtsteile GDMA gerührt wurden.

### Herstellungsbeispiel 4 (CuDMA-Lösung)

Es wurde eine homogene CuDMA-Lösung hergestellt indem 2 Gewichtsteile CuDMA mithilfe eines Magnetrührers in 98 Gewichtsteile HEMA gerührt wurden.

### Herstellungsbeispiel 5 (TMBSNa)

TMBSNa (Pulver) wurde gemäß Beispiel 1 der WO 2008/000313 A1 hergestellt.

### Herstellungsbeispiel 6 (PBSNa)

Zunächst wurden 50 g PBS in 450 g Dioxan gelöst, dann wurden 9,2 g Natrium-Methanolat, gelöst in 100 g Methanol, unter Rühren hinzugetropft. Der Ansatz wurde 3 Stunden bei 100°C gerührt. Nach dem Abkühlen wurde das ausgefallene PBSNa abfiltriert, mit Dioxan gewaschen und anschließend getrocknet.

Es wurden jeweils zwei Pasten (Komponenten A und B) hergestellt. Die Beispielzusammensetzungen sind in Tabelle 1 wiedergegeben.

Die in Tabelle 1 angegebenen Komponenten wurden hergestellt indem zunächst die flüssigen Verbindungen und Lösungen in einem Glasbehälter homogen vermischt wurden. In diese flüssigen Gemische wurde, sofern in der Komponente vorgesehen, BPO gemäß Tabelle 1 eingewogen. Die so erstellten Mischungen wurden mithilfe eines Überkopfrührers solange gerührt bis eine klare Lösung vorlag.

Die homogenen flüssigen Mischungen wurden quantitativ in einen Vakuumlabormischer (Fa. PC-Laborsystems, Schweiz) überführt. R812S und BAF1,5sil. als Füllstoffe wurden portionsweise zugegeben und bis zur homogenen Verteilung im Gemisch gerührt.

Die so hergestellten Pasten wurden über einen Dreiwalzenstuhl (Fa. Exakt, Norderstedt, Deutschland) zur weiter verbesserten Dispergierung der Füllstoffe gewalzt, wieder in den Vakuummischer überführt und dort evakuiert.

**Tabelle 1**

| **Komponente A** | | | | | | | | | |
|---|---|---|---|---|---|---|---|---|---|
| **Beispiel** | **Ref. 1** | **Ref. 2** | **Ref. 3** | **Ref. 4** | **Ref. 5** | **1** | **2** | **3** | **4** |
| | [%] | [%] | [%] | [%] | [%] | [%] | [%] | [%] | [%] |
| Harz45/55 | 23,03 | 22,86 | 12,24 | 18,49 | 22,90 | 26,65 | 22,90 | 22,90 | 22,90 |
| UDMA | 13,82 | 13,72 | 7,35 | 11,10 | 13,74 | 15,99 | 13,74 | 13,74 | 13,74 |
| GDMA | 1,57 | 1,34 | 0,00 | 1,33 | 1,57 | 1,57 | 1,57 | 1,57 | 1,57 |
| BTBACl-Lösun | - | 0,44 | 2,96 | 0,44 | - | - | - | - | - |
| DHBS-Lösung | - | - | 13,05 | 13,06 | - | - | - | - | - |
| CuDMA-Lösung | - | 0,12 | - | - | - | - | - | - | - |
| MMHE | 6,00 | 6,00 | - | - | - | - | 6,00 | 6,00 | - |
| HEAP | - | - | - | - | 6,00 | - | - | - | 6,00 |
| BPO | - | - | - | - | 2,00 | 2,00 | 2,00 | 2,00 | 2,00 |
| BAF 1,5 sil. | 52,10 | 52,02 | 60,92 | 52,10 | 50,31 | 50,31 | 50,31 | 50,31 | 50,31 |
| R812S | 3,48 | 3,50 | 3,48 | 3,48 | 3,49 | 3,49 | 3,49 | 3,49 | 3,49 |
| | 100,00 | 100,00 | 100,00 | 100,00 | 100,00 | 100,00 | 100,00 | 100,00 | 100,00 |

| **Komponente B** | | | | | | | | | |
|---|---|---|---|---|---|---|---|---|---|
| **Beispiel** | **Ref. 1** | **Ref. 2** | **Ref. 3** | **Ref. 4** | **Ref. 5** | **1** | **2** | **3** | **4** |
| | [%] | [%] | [%] | [%] | [%] | [%] | [%] | [%] | [%] |
| Harz 45/55 | 27,44 | 27,44 | 27,44 | 27,44 | 27,44 | 27,44 | 27,44 | 26,88 | 26,88 |
| UDMA | 16,47 | 16,47 | 16,47 | 16,47 | 16,47 | 16,47 | 16,47 | 16,13 | 16,13 |
| HEMA | 0,30 | 0,30 | - | 0,18 | 0,30 | 0,30 | 0,30 | 0,18 | 0,18 |
| CuDMA-Lösung | - | - | 0,30 | 0,12 | - | - | - | 0,12 | 0,12 |
| TMBSNa | - | - | 1,39 | 1,39 | - | - | - | - | - |
| PBSNa | 2,39 | 2,39 | - | - | - | 2,39 | 2,39 | 2,39 | 2,39 |
| N,N-Bis | - | - | - | - | 2,39 | - | - | - | - |
| Aminoester | - | - | - | - | - | - | - | 2,00 | 2,00 |
| BAF 1,5 sil. | 49,92 | 49,92 | 50,92 | 50,92 | 49,92 | 49,92 | 49,92 | 48,90 | 48,90 |
| R812S | 3,48 | 3,48 | 3,48 | 3,48 | 3,48 | 3,48 | 3,48 | 3,41 | 3,41 |
| | 100,00 | 100,00 | 100,00 | 100,00 | 100,00 | 100,00 | 100,00 | 100,00 | 100,00 |

| **Messwerte** | | | | | | | | | |
|---|---|---|---|---|---|---|---|---|---|
| **Beispiel** | **Ref. 1** | **Ref. 2** | **Ref. 3** | **Ref. 4** | **Ref. 5** | **1** | **2** | **3** | **4** |
| EHZ [s] | | 1964 | 516 | 299 | 302 | 21 | 207 | 64 | 191 |
| SHV [min] | >720 | 26 | 5 | 35 | 10 | 2 | 4 | 1,5 | 3 |
| BF [MPa] | | 33±3 | 46±4 | 48±5 | 125±18 | 97±5 | 88±1 | 112±7 | 109±8 |
| BM [MPa] | | 960±90 | 1540±180 | 1540±100 | 4690±430 | 3490±330 | 2050±10 | 4500±280 | 3760±370 |
| ΔE(37°C,15 Tage) | | | | | 13,48 | | | | 1,99 |
| ΔE(37°C,30 Tage) | | | | | 16,76 | | | | 2,35 |
| ΔE(60°C,15 Tage) | | | | | 27,12 | | | | 3,22 |
| ΔE(60°C,30 Tage) | | | | | 34,63 | | | | 4,81 |

Die Pasten A und B wurden jeweils paarig innerhalb eines Beispiels untersucht.

### Bestimmung von Biegefestigkeit (BF) und Biegemodul (BM):

Die jeweiligen Komponenten A und B einer Beispielzusammensetzung wurden blasenfrei in die Kammern einer 25 ml Sicherheitsdoppelkartusche (CS025-01-13, Fa. MixPac) eingefüllt. Gemäß ISO 4049 wurden durch automatische Anmischung (Mischkanüle MB4.2-12D, Fa. MixPac) je Beispielzusammensetzung (Materialkombination) 5 Prüfkörper hergestellt, gelagert und vermessen.

### Bestimmung des sensorischen Erhärtungsverhaltens (SHV):

Die jeweiligen Komponenten A und B einer Beispielzusammensetzung wurden mithilfe eines Mischspatels (Fa. DMG) auf einem handelsüblichen Mischblock (Fa. DMG) angemischt und der Verlauf der Erhärtung durch manuelles prüfen der Festigkeit der angemischten Pasten im Abstand von 15 s geprüft, bis die jeweilige Mischung einen sensorisch harten, ausgehärteten Zustand erreicht hatte. In Tabelle 1 ist die Zeit vom Beginn des Mischvorgangs bis zum Zeitpunkt an dem der sensorisch harte, ausgehärtete Zustand erreicht wurde, angegeben. Die Pasten der Referenzbeispiele 1, 3 und 5 gingen nach ca. 5 min in einen gelartigen Zustand über.

### Bestimmung der Enderhärtungszeit (EHZ):

Die Enderhärtungszeit (EHZ) der gemischten Pasten wurde im Rheometer mit koaxialen Zylindern ermittelt. Jeweils eine etwa konstante Pastenmenge wurde nach, auch für die Messung von Biegefestigkeit und -modul verwendeter, automatischer Anmischung auf einen unteren Kunststoffzylinder gegeben. 60s (Beispiel 2: 30s) nach Mischbeginn wurde ein permanent auf 37°C temperierter oberer Metallhohlzylinder bis zu einem definierten Abstand zum unteren Zylinder in die Paste gedrückt. Die beiden Zylinder wurden oszillierend gegeneinander bewegt und die hierfür erforderliche Kraft wurde mit Hilfe eines Schreibers aufgezeichnet. Die Enderhärtungszeit war diejenige Zeit, ab der die aufgezeichnete Kraft konstant blieb.

### Bestimmung der Farbstabilität (ΔE):

Die Komponenten A und B der Beispiele wurden im Verhältnis 1:1 gemischt und in zylindrischen Formen (Ø 15 mm, 1 mm hoch) bei Raumtemperatur ausgehärtet. Die Farbe der resultierenden Materialzylinder wurde mithilfe eines Farbmessgeräts (Fa. Datacolor) im CIELAB-System bestimmt. Anschließend wurden die Materialzylinder in Wasser bei 37°C oder 60°C gelagert und nach verschiedenen Lagerzeiten erneut die Farbe mithilfe des Farbmessgeräts im CIELAB-System bestimmt. Angegeben wird die Farbveränderung (ΔE) jeweils im Vergleich zur Ausgangsfarbmessung. Farbveränderungen mit ΔE < 3 werden im Allgemeinen als nicht optisch wahrnehmbar bewertet.

### Erläuterung der Beispiele und Referenzbeispiele

**Referenzbeispiel 1** zeigt, dass Mischungen einer Komponente enthaltend bei Raumtemperatur polymerisierbare säuregruppenfreie und bei Raumtemperatur polymerisierbare säuregruppenhaltige Monomere mit einer Komponente enthaltend bei Raumtemperatur polymerisierbare säuregruppenfreie Monomere und enthaltend das Salz einer CH-aciden Verbindung nicht ausreichend schnell polymerisieren und somit klinisch nicht anwendbar sind, sofern Sie nicht weitere nicht polymerisierbare Säuren enthalten.

**Referenzbeispiel 2** zeigt, dass sich das Erhärtungsverhalten durch Zugabe von Akzeleratoren wie Cu(II)-Verbindungen und Chlorid leicht verbessert, aber noch nicht zu klinisch verwendbaren Materialien führt.

Die **Referenzbeispiele 3 und 4** zeigen, dass die Verwendung nicht polymerisierbarer Säuren mit Cu(II)-Verbindungen und Chlorid zu verbessertem Erhärtungsverhalten gegenüber der Verwendung polymerisierbarer Säuren führt, die mechanischen Eigenschaften sind jedoch weiterhin verbesserungsbedürftig.

**Referenzbeispiel 5** zeigt, dass die Verwendung von BPO/Amin mit den säuregruppenhaltigen Monomeren zu gutem Erhärtungsverhalten und verbesserten mechanischen Eigenschaften führt, aber mit einer schlechte Farbstabilität einhergeht.

**Beispiel 1** zeigt eine sehr kurze Erhärtungszeit (EHZ)und eine gute Biegefestigkeit (BF) sowie ein gutes Biegemodul (BM), gegenüber den Referenzbeispielen 2, 3 und 4 war die Biegefestigkeit (BF) sowie des Biegemoduls (BM) bei vergleichbarer Farbstabilität verbessert, gegenüber dem Referenzbeispiel 5 (BPO/Amin) war die Farbstabilität wesentlich verbessert. (Die Farbstabilitäten der Referenzbeispiele 1-4 sowie der Beispiele 1-4 waren sehr ähnlich.)

**Beispiel 2** zeigt, dass die Zugabe einer Säure (Maleinsäure-Mono-2-Methacryloyloxyethylester) keine weitere Verbesserung von EZ, BF und BM gegenüber dem Beispiel 1 (BPO/PBSNa ohne Säure) bewirkte, sondern tendenziell vergleichbare Werte.

**Beispiele 3 und 4** zeigen weitere Verbesserungen des Systems BPO/PBSNa durch Kombination mit Cu^{II}/Cl⁻ und/oder basischen Monomeren wie N,N-Dimethylaminoethylmethacrylat. In Beispiel 4 ist die relativ schwache Carboxylsäuregruppe durch die starke Phosphorsäuregruppe ersetzt, liefert jedoch ebenso sehr gute Werte. Dies zeigt die verbesserte Säuretoleranz des Initiatorystems.

Weiterhin zeigen diese Beispiele, dass sich die das CH-acide Salz enthaltende Komponente weiter stabilisieren lässt, wenn sie basische Verbindungen enthält. Bevorzugt sind die basischen Verbindungen Monomere enthaltend basische Gruppen. Materialen, bei denen die die CH-aciden Salze enthaltende Komponente auch basische Monomere enthält, zeigen bei gutem Erhärtungsverhalten und hervorragenden mechanischen Eigenschaften überraschend auch eine sehr gute Farbstabilität.

## Patentansprüche

1. Polymerisierbares Dentalmaterial aus wenigstens zwei Komponenten, **dadurch gekennzeichnet, dass** es die folgenden Komponenten umfasst:
Komponente A enthaltend:
i) radikalisch polymerisierbare Monomere ohne Säuregruppe,
ii) wenigstens ein Peroxid der Formel (1) 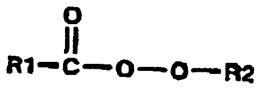 mit
R1= H, Alkyl, Aryl, Alkoxy, Aryloxy, oder (R3)(R3)N-; R2, R3 gleich oder verschieden ausgewählt aus der Gruppe bestehend aus C(O)-R1, S(O₂)-R1, Alkyl und Aryl.
Komponente B enthaltend:
iii) radikalisch polymerisierbare Monomere ohne Säuregruppe,
iv) das Salz einer CH-aciden Verbindung, wobei die CH-acide Verbindung eine radikalische Polymerisation auslösen kann.

2. Polymerisierbares Dentalmaterial nach Anspruch 1, **dadurch gekennzeichnet, dass** die Komponente B keine das Salz der CH-aciden Verbindung protonierende Säure enthält.

3. Polymerisierbares Dentalmaterial nach Anspruch 1 oder 2, **dadurch gekennzeichnet, dass** R1 Alkyl, Aryl, Alkoxy oder Aryloxy ist.

4. Polymerisierbares Dentalmaterial nach Anspruch 3, **dadurch gekennzeichnet, dass** R2 gleich C(O)-R1 ist.

5. Polymerisierbares Dentalmaterial nach einem der Ansprüche 1 bis 4, **dadurch gekennzeichnet, dass** die Komponente A zusätzlich radikalisch polymerisierbare Monomere mit Säuregruppe enthält.

6. Polymerisierbares Dentalmaterial nach einem der Ansprüche 1 bis 5, **dadurch gekennzeichnet, dass** die Komponente B zusätzlich radikalisch polymerisierbare Monomere mit basischer Gruppe enthält, bevorzugt 0,2 bis 5 Gew.-%, weiter bevorzugt 0,5 bis 4 Gew.-%, weiter bevorzugt 1 bis 3 Gew.-%.

7. Polymerisierbares Dentalmaterial nach einem der Ansprüche 1 bis 6, **dadurch gekennzeichnet, dass** der Harzanteil des polymerisierbaren Dentalmaterials insgesamt über 50 Gew.-%, vorzugsweise über 60 Gew.-%, weiter vorzugsweise über 70 Gew.-%, weiter vorzugsweise über 80 Gew.-%, weiter vorzugsweise über 90 Gew.-%, weiter vorzugsweise über 95 Gew.-%, weiter vorzugsweise über 98 Gew.-% radikalisch polymerisierbare Monomere enthält.

8. Polymerisierbares Dentalmaterial nach einem der Ansprüche 1 bis 7, **dadurch gekennzeichnet, dass** das Salz der CH-aciden Verbindung der Komponente B ein Salz der α-Benzoylproprionitrile, α-Cyancarbonsäureester und -amide, cyclischer β-Oxonitrile, β-Diketone, cyclischer β-Diketone, cyclischer β-Oxocarbonsäureester, cyclischer β-Oxolactone, Malonsäure, Malonsäurederivate, Pyrazolderivate, Barbitursäure, Thiobarbitursäure oder Barbitursäure oder Thiobarbitursäurederivate ist.

9. Polymerisierbares Dentalmaterial nach einem der Ansprüche 1 bis 8, **dadurch gekennzeichnet, dass** das Salz der CH-aciden Verbindung ein Salz ausgewählt aus der Gruppe bestehend aus einwertigen und zweiwertigen Salzen der Alkali- und Erdalkaliionen, vorzugsweise Natrium, der Stickstoffkationen oder der Phosphorkationen ist.

10. Polymerisierbares Dentalmaterial nach einem der Ansprüche 1 bis 9, **dadurch gekennzeichnet, dass** das Salz der CH-aciden Verbindung ein Salz ausgewählt aus der Gruppe bestehend aus Ammoniumionen der Formel NR₄⁺, oder Phosphoniumionen der Formel PR₄⁺ ist, wobei R gleich oder verschieden sein kann und ein H-Atom, eine Alkylgruppe, eine Arylgruppe oder eine Aralkylgruppe, wie z.B. eine Benzylgruppe, darstellt.

11. Polymerisierbares Dentalmaterial nach einem der Ansprüche 1 bis 9, **dadurch gekennzeichnet, dass** mindestens eine der Komponenten des polymerisierbaren Dentalmaterials einen Füllstoff enthält, ausgewählt aus der Gruppe bestehend aus mikro- und/oder nanoskaligen Füllstoffen, vorzugsweise Metall-, Halbmetall- oder Mischmetalloxide, Silikaten, Nitriden, Sulfaten, Titanaten, Zirkonaten, Stannaten, Wolframaten, Siliciumdioxiden oder einer Mischung aus diesen Verbindungen oder sphärischen Füllstoffen, Quarzpulvern, Glaspulvern, Glaskeramikpulvern oder eine Mischung aus diesen Pulvern oder gefüllten oder ungefüllten Splitterpolymerisaten und/oder Perlpolymerisaten.

12. Polymerisierbares Dentalmaterial nach Anspruch 10, **dadurch gekennzeichnet, dass** der Füllstoff ein oberflächenmodifizierter Füllstoff, vorzugsweise eine organisch oberflächenmodifizierter Füllstoff, weiter vorzugsweise ein silanisierter Füllstoff ist.

13. Polymerisierbares Dentalmaterial nach einem der Ansprüche 10 bis 12, **dadurch gekennzeichnet, dass** der Füllstoffgehalt des Materials insgesamt 10 bis 90 Gew.-%, weiter vorzugsweise 20 bis 80 Gew.-%, weiter vorzugsweise 30 bis 70 Gew.-%, weiter vorzugsweise 40 bis 60 Gew.-% beträgt.

14. Polymerisierbares Dentalmaterial nach einem der Ansprüche 1 bis 13 zur Verwendung als Füllungsmaterial, Stumpfaufbaumaterial, Befestigungsmaterial, Bondingmaterial, provisorisches und permanentes Kronen- und Brückenmaterial, zahntechnischen Werkstoff zur Herstellung von Inlays, Onlays, Verblendschalen, künstlichen Zähnen, Modellmaterialien und Fissuren- bzw. Wurzelkanal-Versiegelungsmaterial.

15. Gehärtetes Dentalmaterial, erhältlich aus einem polymerisierbaren Dentalmaterial nach einem der Ansprüche 1 bis 13.

## Claims

1. A polymerisable dental material prepared from at least two components, **characterised in that** it comprises the following components:
component A containing:
i) free-radically polymerisable monomers without an acid group,
ii) at least one peroxide of formula (1) 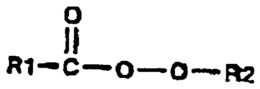 with
R1 being H, alkyl, aryl, alkoxy, aryloxy, or (R3)(R3)N-;
R2, R3, identical or different, being selected from the group consisting of C(O)-Rl, S(O₂)-R1, alkyl and aryl;
component B containing:
iii) free-radically polymerisable monomers without an acid group,
iv) the salt of a CH-acidic compound, the CH-acidic compound being capable of initiating free-radical polymerisation.

2. A polymerisable dental material according to claim 1, **characterised in that** component B does not contain an acid which protonates the salt of the CH-acidic compound.

3. A polymerisable dental material according to claim 1 or claim 2, **characterised in that** R1 is alkyl, aryl, alkoxy or aryloxy.

4. A polymerisable dental material according to claim 3, **characterised in that** R2 is identical to C(O)-R1.

5. A polymerisable dental material according to any one of claims 1 to 4, **characterised in that** component A additionally contains free-radically polymerisable monomers with an acid group.

6. A polymerisable dental material according to any one of claims 1 to 5, **characterised in that** component B additionally contains free-radically polymerisable monomers with a basic group, preferably 0.2 to 5 wt.%, more preferably 0.5 to 4 wt.%, more preferably 1 to 3 wt.%.

7. A polymerisable dental material according to any one of claims 1 to 6, **characterised in that** the resin content of the polymerisable dental material contains in total greater than 50 wt.%, preferably greater than 60 wt.%, more preferably greater than 70 wt.%, more preferably greater than 80 wt.%, more preferably greater than 90 wt.%, more preferably greater than 95 wt.%, more preferably greater than 98 wt.% of free-radically polymerisable monomers.

8. A polymerisable dental material according to any one of claims 1 to 7, **characterised in that** the salt of the component B CH-acidic compound is a salt of α-benzoylpropionitrile, α-cyanocarboxylic acid esters and amides, cyclic β-oxonitriles, β-diketones, cyclic β-diketones, cyclic β-oxocarboxylic acid esters, cyclic β-oxolactones, malonic acid, malonic acid derivatives, pyrazole derivatives, barbituric acid, thiobarbituric acid or barbituric acid or thiobarbituric acid derivatives.

9. A polymerisable dental material according to any one of claims 1 to 8, **characterised in that** the salt of the CH-acidic compound is a salt selected from the group consisting of monovalent and divalent salts of alkali metal and alkaline earth metal ions, preferably sodium, of nitrogen cations or of phosphorus cations.

10. A polymerisable dental material according to any one of claims 1 to 9, **characterised in that** the salt of the CH-acidic compound is a salt selected from the group consisting of ammonium ions of formula NR₄⁺, or phosphonium ions of formula PR₄⁺, wherein R may be identical or different and represents an H atom, an alkyl group, an aryl group or an aralkyl group, such as for example a benzyl group.

11. A polymerisable dental material according to any one of claims 1 to 9, **characterised in that** at least one of the components of the polymerisable dental material contains a filler selected from the group consisting of micro- and/or nanoscale fillers, preferably metal, semimetal or mischmetal oxides, silicates, nitrides, sulfates, titanates, zirconates, stannates, tungstates, silicon dioxides or a mixture of these compounds or spherical fillers, quartz powders, glass powders, glass-ceramic powders or a mixtures of these powders or filled or unfilled splinter polymers and/or bead polymers.

12. A polymerisable dental material according to claim 10, **characterised in that** the filler is a surface-modified filler, preferably an organically surface-modified filler, more preferably a silanised filler.

13. A polymerisable dental material according to any one of claims 10 to 12, **characterised in that** the filler content of the material amounts in total to 10 to 90 wt.%, more preferably 20 to 80 wt.%, more preferably 30 to 70 wt.%, more preferably 40 to 60 wt.%.

14. A polymerisable dental material according to any one of claims 1 to 13 for use as a filling material, core build-up material, fastening material, bonding material, temporary and permanent crown and bridge material, dental material for producing inlays, onlays, veneers, artificial teeth, modelling materials and fissure or root canal sealant material.

15. A cured dental material obtainable from a polymerisable dental material according to any one of claims 1 to 13.

## Revendications

1. Matériel dentaire polymérisable constitué d'au moins deux composants, **caractérisé en ce qu'**il comprend les composants suivants :
composant A contenant :
i) des monomères polymérisables au niveau radicalaire sans groupe acide,
ii) au moins un peroxyde de formule (1) 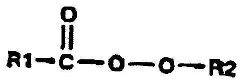
où
R1 = H, un groupe alkyle, aryle, alcoxy, aryloxy ou (R3) (R3)N- ;
R2, R3, identiques ou différents, choisis dans le groupe comprenant C(O)-R1, S(O₂)-R1, un groupe alkyle et aryle.
Composant B contenant :
iii) des monomères polymérisables au niveau radicalaire sans groupe acide,
iv) le sel d'un composé CH-acide, le composé CH-acide pouvant déclencher une polymérisation radicalaire.

2. Matériau dentaire polymérisable selon la revendication 1, **caractérisé en ce que** le composant B ne contient pas de sel de l'acide protonant du composé CH-acide.

3. Matériau dentaire polymérisable selon la revendication 1 ou 2, **caractérisé en ce que** R1 est un groupe alkyle, aryle, alcoxy ou aryloxy.

4. Matériau dentaire polymérisable selon la revendication 3, **caractérisé en ce que** R2 est semblable à C(O)-R1.

5. Matériau dentaire polymérisable selon l'une des revendications 1 à 4, **caractérisé en ce que** le composant A contient en outre des monomères polymérisables au niveau radicalaire avec des groupes acides.

6. Matériau dentaire polymérisable selon l'une des revendications 1 à 5, **caractérisé en ce que** le composant B contient en outre des monomères polymérisables au niveau radicalaire avec des groupes basiques, de préférence 0,2 à 5 % massique, mieux 0,5 à 4 % massique, mieux encore 1 à 3 % massique.

7. Matériau dentaire polymérisable selon l'une des revendications 1 à 6, **caractérisé en ce que** la partie de résine du matériau dentaire polymérisable contient au total plus de 50 % massique, de préférence plus de 60 % massique, mieux plus de 70 % massique, mieux encore plus de 80 % massique, mieux encore plus de 90 % massique, mieux encore plus de 95 % massique, mieux encore plus de 98 % massique de monomères polymérisables au niveau radicalaire.

8. Matériau dentaire polymérisable selon l'une des revendications 1 à 7, **caractérisé en ce que** le sel du composé CH-acide du composant B est un sel d'α-benzoylproprionitrile, d'ester et d'amide d'acide α-cyanocarboxylique, de β-oxonitrile cyclique, de β-dicétone, de β-dicétone cyclique, d'ester d'acide β-oxocarboxylique cyclique, de β-oxolactone cyclique, d'acide malonique, de dérivé d'acide malonique, de dérivé de pyrazol, d'acide barbiturique, d'acide thiobarbiturique ou de dérivés d'acide barbiturique ou thiobarbiturique.

9. Matériau dentaire polymérisable selon l'une des revendications 1 à 8, **caractérisé en ce que** le sel du composé CH-acide est un sel choisi dans le groupe consistant en sels monovalents et bivalents d'ions alcalins et alcalino-terreux, de préférence de sodium, de cations d'azote ou de cations de phosphore.

10. Matériau dentaire polymérisable selon l'une des revendications 1 à 9, **caractérisé en ce que** le sel du composé CH-acide est un sel choisi dans le groupe consistant en ions ammonium de formule NR₄⁺ ou en ions phosphonium de formule PR₄⁺, les R pouvant être identiques ou différents et représentant un atome H, un groupe alkyle, un groupe aryle ou un groupe aralkyle, tel que par exemple, un groupe benzyle.

11. Matériau dentaire polymérisable selon l'une des revendications 1 à 9, **caractérisé en ce qu'**au moins l'un des composants du matériau dentaire polymérisable contient un agent de charge choisi dans le groupe consistant en agents de charge à l'échelle du micron et/ou à l'échelle du nanomètre, de préférence un oxyde de métal, de semi-métal ou de mischmétal, des silicates, des nitrures, des sulfates, des titanates, des zirconates, des stannates, des wolframates, des dioxydes de silicium ou un mélange de ces composés ou des agents de charge sphériques, des poudres de quartz, des poudres de verre, des poudres de vitrocéramique ou un mélange de ces poudres ou des polymères à éclats et/ou des polymères nacrés remplis ou non remplis.

12. Matériau dentaire polymérisable selon la revendication 10, **caractérisé en ce que** l'agent de charge est un agent de charge modifié en surface, de préférence un agent de charge modifié en surface organiquement, mieux encore un agent de charge silanisé.

13. Matériau dentaire polymérisable selon l'une des revendications 10 à 12, **caractérisé en ce que** la teneur en agent de charge du matériau est au total de 10 à 90 % massique, de préférence de 20 à 80 % massique, mieux de 30 à 70 % massique, mieux encore de 40 à 60 % massique.

14. Matériau dentaire polymérisable selon l'une des revendications 1 à 13, pour l'utilisation comme matériau de comblement, matériau de constitution de moignon, matériau de consolidation, matériau de scellement, matériau de couronne et de bridge provisoire et permanent, matériau de technique dentaire pour la production d'inlays, d'onlays, de facettes, de dents artificielles, de matériaux de modèle et de matériau d'obturation de fissures ou de canal radiculaire.

15. Matériau dentaire durci, pouvant être obtenu à partir d'un matériau dentaire polymérisable selon l'une des revendications 1 à 13.
